# EUROPÄISCHE PATENTSCHRIFT

(11) **EP 0 403 876 B1**
(45) Veröffentlichungstag und Bekanntmachung des Hinweises auf die Patenterteilung: **13.04.1994**
(21) Anmeldenummer: 90110689.8
(22) Anmeldetag: 06.06.1990
(51) Int. Cl.: G01N 31/22, G01N 33/84, C12Q 1/58

(54) **Verfahren zur Bestimmung der Gebrauchsintensität von Textilstücken und Vorrichtung zur Durchführung des Verfahrens**
Method and device for determining the use intensity of textile pieces
Procédé et dispositif pour déterminer l'intensité d'utilisation de pièces textiles

(30) Priorität: 19.06.1989 DE 3919946
(43) Veröffentlichungstag der Anmeldung: 27.12.1990
(73) Patentinhaber: H. Brinkhaus GmbH & Co. KG, D-48231 Warendorf (DE)
(72) Erfinder: Ende, Klaus-Dieter, D-4410 Warendorf 1 (DE); Brokmann, Kurt, D-4410 Warendorf 1 (DE); Cammann, Karl, Prof., Dr., D-4400 Münster (DE)
(74) Vertreter: Hoffmeister, Helmut, Dr. Dipl.-Phys.

(56) Entgegenhaltungen:
- EP-A- 0 005 519
- WO-A-87/00744
- CH-A- 465 916
- DE-U- 7 708 156
- US-E- 30 267

## Beschreibung

Die Erfindung betrifft ein Verfahren zur Bestimmung der Gebrauchsintensität von Textilstücke, z. B. Kleidungsstücken oder Bettwäsche, und eine Vorrichtung zur Durchführung des Verfahrens

Textilstücke, z. B. Bekleidungsstücke oder Bettwäsche, bedürfen nach einer gewissen Benutzung der Reinigung. Unter dem Begriff Bettwäsche werden hier auch mit Daunen, Federn oder einem anderen Füllmaterial gefüllte Oberbetten und Kopfkissen verstanden. Teilweise werden z. B. in Hotels als Ersatz hierfür auch mit Bezügen versehene Wolldecken eingesetzt. In vielen Fällen tritt die Notwendigkeit einer Reinigung der genannten Textilstücke ein, ohne daß es zu einer sichtbaren Verschmutzung kommt. Insbesondere, wenn der Benutzer stark schwitzt, kann es notwendig sein, die genannten Textilstücke zu reinigen, bevor sie äußerlich sichtbar verschmutzt sind. Es ist wünschenswert, in solchen Fällen den Zeitpunkt einer notwendigen Reinigung bestimmen zu können, bevor das Textilstück unangenehm riecht.

Andererseits werden z. B. in Hotels, Jugendherbergen, Krankenhäusern oder anderen Beherbungsstätten die Oberbetten und Kopfkissen nach einem festgelegten Zeitplan gereinigt. Dies kann im Einzelfall bedeuten, daß das festgelegte Reinigungsintervall zu lang bemessen ist oder in anderen Fällen eine Reinigung durchgeführt wird, bevor sie tatsächlich notwendig ist. Auch hier ist es also aus hygienischen und wirtschaftlichen Gründen wünschenswert, den Zeitpunkt einer notwendigen Reinigung möglichst genau und ohne großen Aufwand bestimmen zu können.

Es wurde nun gefunden, daß durch die Bestimmung der an ein Textilstück abgegebenen Schweißmenge der Reinigungszeitpunkt festgestellt werden kann.

Der Erfindung liegt daher die Aufgabe zugrunde, ein Verfahren und eine Vorrichtung zu schaffen, die die Bestimmung der Gebrauchsintensität von Textilstücken ermöglicht.

Diese Aufgabe wird erfindungsgemäß dadurch gelöst, daß wenigstens eine einen Farbumschlag bei Reaktion mit einer für den menschlichen Schweiß charakteristischen Substanz erzeugende Indikatorsubstanz verwendet wird. Anhand des durch den Schweiß ausgelösten Farbumschlags läßt sich so leicht feststellen, ob ein Textilstück gereinigt werden muß oder nicht.

Vorteilhaft dient der im menschlichen Schweiß enthaltene Harnstoff als die den Farbumschlag auslösende Substanz. Andererseits kann auch freies Ammoniak (NH3) als eine den Farbumschlag erzeugende Substanz verwendet werden. NH3 entsteht im menschlichen Körper zwar nur als pathologisches Stoffwechselprodukt; es wird jedoch auch durch natürliche Zersetzung von Harnstoff außerhalb und innerhalb des Körpers NH3 frei, so daß auch Ammoniak als Indikator verwendbar ist.

Eine unter den genannten Umständen einfache Bestimmungsmethode ergibt sich dadurch, daß als Indikatorsubstanz für Harnstoff eine Mischung aus Urease und einem pH-Wert-Indikator verwendet wird. Durch die Urease erfolgt eine fermentative Zersetzung des Harnstoffs in Kohlendioxid und Ammoniak. Die pH-Wert-Indikator ermöglicht eine einfache Anzeige des freigesetzten Ammoniaks, was, wie gesagt, auch durch natürliche Zersetzung des Harnstoffes freiwird.

Als pH-Wert-Indikatoren werden vorteilhaft Phenolphthalein oder Lackmus verwendet. Bei dem erfindungsgemäßen Verfahren wird der Schweiß also dadurch angezeigt, daß er zunächst durch die Einwirkung der Urease zersetzt wird und das entstandene Spaltprodukt Ammoniak einen Farbumschlag des pH-Wert-Indikators auslöst. Das Verfahren läßt sich leicht durchführen und ermöglicht eine zuverlässige Bestimmung der Gebrauchsintensität von Textilstücken.

Um einen Zeitfaktor frei wählen zu können, wird vorgeschlagen, daß sich das freie Ammoniak zunächst an eine saure Puffersubstanz bindet und erst nach Neutralisierung der Puffersubstanz sich der Farbumschlag ergibt. Als saure Puffsubstanz kann beispielsweise Citronensäure, Weinsteinsäure oder dergleichen dienen.

Die Erfindung betrifft auch eine Vorrichtung zur Durchführung des Verfahrens, die dadurch gekennzeichnet ist, daß sie als Knopf, Fahne oder dergleichen ausgebildet ist, mit einem Textilstück, z. B. einem Bekleidungsstück oder Bettwäsche, verbindbar ist und wenigstens eine einen Farbumschlag bei Reaktion mit einer für den menschlichen Schweiß charakteristischen Substanz erzeugende Indikatorsubstanz trägt.

Vorteilhafte Ausgestaltungen der erfindungsgemäßen Vorrichtung ergeben sich aus den weiterfen Unteransprüchen.

Soweit die Indikatorsubstanzen wasserlöslich sind oder durch Reinigungsmittel ausgewaschen werden, ergibt sich die Notwendigkeit, die Vorrichtung nach einer Reinigung zu ersetzen. Dies kann in einfacher Weise dadurch geschehen, daß ein neuer Knopf oder eine neue Fahne z. B. in Form eines Stoffabschnitts, der die Indikatorsubstanzen trägt, an den Textilstück durch Annähen oder Anknöpfen befestigt werden.

Bei einer besonders vorteilhaften Ausgestaltung der erfindungsgemäßen Vorrichtung ist die Indikatorsubstanz oder sind die Indikatorsubstanzen in durch Druck zerstörbare Mikrokapseln eingeschlossen. Hierdurch wird es ermöglicht, zur Prüfung der Gebrauchsintensität nur einen Teil der Mikrokapseln zu zerstören, um festzustellen, ob eine Reinigung erforderlich ist. Die Mikrokapseln sind so gestaltet, daß sie von wässrigen oder anderen Reinigungsmitteln nicht angegriffen werden. Die bei der Prüfung nicht zerstörten Mikrokapseln können also nach einer erfolgten Reinigung zur erneuten Prüfung dienen. Hierdurch entfällt die Notwendigkeit, nach jeder Reinigung die Vorrichtung zu ersetzen.

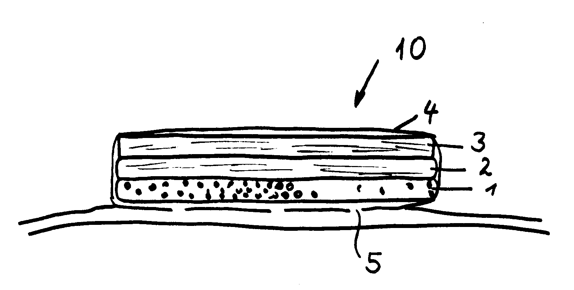

Im folgenden wird die Erfindung anhand zweier Ausführungsbeispiele näher erläutert (vgl. auch die Figur).

### Beispiel 1:

0,5 g Urease werden in 50 ml Wasser unter Schütteln gelöst. Zu der Lösung werden 40 Tropfen einer Phenolphthaleinlösung hinzugegeben. Mit der erhaltenen Lösung werden rechteckige Leinenabschnitte von 3 x 8 cm² Größe getränkt und an der Luft getrocknet. Die so vorbereiteten Stoffabschnitte werden an einem Oberbett, einem Kopfkissen, an der Innenseite eines Pullovers und eines Mantels befestigt.

Je nach Intensität der Schweißabgabe des jeweiligen Benutzers tritt nach unterschiedliche Benutzungsdauer eine Rotfärbung der Stoffabschnitte auf. Sie zeigt an, daß das Bettzeug bzw. die Kleidungsstücke gereinigt werden sollten.

### Beispiel 2:

### (vgl. Figur)

Ein erster Zellstoff-Streifen 3 von etwa 5 mm Dicke wird mit einer Lackmus-Lösung getrankt und getrocknet. Ein zweiter Zellstoff-Streifen wird mit einer wässrigen, gesättigten Lösung von Citronensäure getränkt und ebenfalls getrocknet. Beide Streifen 3 und 2 werden Rücken an Rücken durch punktuell verteilten Klebstoff verbunden. Ein dritter Streifen aus Zellstoff, Bezugszahl 1, wird mit einem neutralen, hydrophilen Mittel getränkt, beispielsweise einem Kristallwasser anziehendes, neutrale Kristall. Derartige "Feuchtstreifen" sind käufliche erhältlich. Der Feuchtstreifen 1 wird mit dem Streifen 2, der die Puffersubstanz tragt, verbunden. Das aus den drei Streifen bestehende Kissen wird in eine durchsichtige Polyethylenfolie eingeschweißt, die auf der Rückseite (also beim Feuchtstreifen) perforiert (bei 5) ist, so daß sie mit der Umgebungsluft in Kontakt treten kann. Das Kissen 10 wird zum Beispiel auf ein Kopfkissen aufgeklebt. Vorhandene Ammoniak-Dämpfe dringen von unten in die Substanz ein und verbinden sich mit der Citronensäure des Kissens 2 bis zur Neutralisation. Erst wenn die Citronensäure verbraucht ist, kommt es zu einer Reaktion mit dem Streifen 3, der mit Lackmus getränkt ist und der sich von Rot nach Blau färbt. Diese Umfärbung kann von der Sichtseite her gesehen werden und bedeutet, daß die vorgegebene Verwendungszeit überschritten ist.

## Patentansprüche

1. Verfahren zur Bestimmung der Gebrauchsintensität von Textilstücken, z. B. Kleidungsstücken oder Bettwäsche, dadurch gekennzeichnet, daß wenigstens eine einen Farbumschlag bei Reaktion mit einer für den menschlichen Schweiß charakteristischen Substanz erzeugende Indikatorsubstanz verwendet wird.

2. Verfahren nach Anspruch 1, dadurch gekennzeichnet, daß der im menschlichen Schweiß enthaltene Harnstoff als die den Farbumschlag auslösende Substanz dient.

3. Verfahren nach Anspruch 1, dadurch gekennzeichnet, daß freies Ammoniak als die den Farbumschlag auslösende Substanz dient.

4. Verfahren nach Anspruch 3, dadurch gekennzeichnet, daß sich das freie Ammoniak zunächst mit einer sauren Puffersubstanz verbindet und daß nach Neutralisation der Puffersubstanz sich der Farbumschlag ergibt.

5. Verfahren nach Anspruch 2, dadurch gekennzeichnet, daß als Indikatorsubstanz eine Mischung aus Urease und einem pH-Wert-Indikator verwendet wird.

6. Verfahren nach Anspruch 4, dadurch gekennzeichnet, daß als pH-Wert-Indikator Phenolphthalein oder Lackmus verwendet werden.

7. Vorrichtung zur Durchführung des Verfahrens nach einem der Ansprüche 1 bis 6, dadurch gekennzeichnet, daß sie als Knopf, Fahne oder dergleichen ausgebildet ist, mit einem Textilstück, z. B. einem Bekleidungsstück oder Bettwäsche, verbindbar ist und wenigstens eine einen Farbumschlag bei Reaktion mit einer für den menschlichen Schweiß charakteristischen Substanz erzeugende Indikatorsubstanz trägt.

8. Vorrichtung nach Anspruch 7, dadurch gekennzeichnet, daß die Indikatorsubstanz oder die Indikatorsubstanzen geeignet sind, bei Reaktion mit Harnstoff einen Farbumschlag zu zeigen.

9. Vorrichtung nach Anspruch 8, gekennzeichnet durch eine Mischung aus Urease und einem pH-Wert-Indikator als Indikatorsubstanzen und durch Phenolphthalein oder Lackmus als pH-Wert-Indikatorsubstanz.

10. Vorrichtung nach einem der Ansprüche 5 bis 9, dadurch gekennzeichnet, daß die Indikatorsubstanz oder die Indikatorsubstanzen in durch Druck zerstörbare Mikrokapseln eingeschlossen sind.

## Claims

1. A method for determining the use intensity of textile pieces, e.g. items of clothing or bed linen, characterised in that at least one indicator substance which produces a colour change upon reacting with a substance characteristic of human sweat is used.

2. A method according to Claim 1, characterised in that the urea contained in human sweat serves as the substance which triggers the colour change.

3. A method according to Claim 1, characterised in that free ammonia serves as the substance which triggers the colour change.

4. A method according to Claim 3, characterised in that the free ammonia initially combines with an acidic buffer substance and that the colour change is produced after neutralisation of the buffer substance.

5. A method according to Claim 2, characterised in that a mixture of urease and a pH value indicator is used as the indicator substance.

6. A method according to Claim 4, characterised in that phenolphthalein or litmus is used as the pH value indicator.

7. A device for performing the method according to one of Claims 1 to 6, characterised in that it is designed as a button, flag or the like, can be joined to a textile piece, e.g. an item of clothing or bed linen, and bears at least one indicator substance which produces a colour change upon reacting with a substance characteristic of human sweat.

8. A device according to Claim 7, characterised in that the indicator substance or the indicator substances are suitable for showing a colour change upon reaction with urea.

9. A device according to Claim 8, characterised by a mixture of urease and a pH value indicator as indicator substances and by phenolphthalein or litmus as a pH value indicator substance.

10. A device according to one of Claims 5 to 9, characterised in that the indicator substance or the indicator substances are enclosed in microcapsules which can be destroyed by pressure.

## Revendications

1. Procédé de détermination de l'intensité d'utilisation de pièces textiles, par exemple des éléments de vêtements ou une literie, caractérisé en ce qu'est utilisé au moins une substance indicatrice engendrant une variation de couleur lors d'une réaction avec une substance caractéristique de la transpiration humaine.

2. Procédé selon la revendication 1, caractérisé en ce que l'urée contenue dans la transpiration humaine sert de substance provoquant la variation des couleurs.

3. Procédé selon la revendication 1, caractérisé en ce que l'ammoniac libre sert de substance déclenchant le changement de couleur.

4. Procédé selon la revendication 3, caractérisé en ce que l'ammoniac libre se combine en premier lieu avec une substance acide de tampon et en ce que le changement de couleur se produit après la neutralisation de la substance de tampon.

5. Procédé selon la revendication 2, caractérisé en ce que l'on utilise comme substance indicatrice un mélange d'uréase et d'un indicateur de la valeur du pH.

6. Procédé selon la revendication 4, caractérisé en ce qu'on utilise de la phtaléine du phénol ou du tournesol comme indicateur de la valeur du pH.

7. Dispositif de mise en oeuvre du procédé selon l'une des revendications 1 à 6, caractérisé en ce qu'il est réalisé sous la forme d'un bouton, d'une carte de marquage ou similaire, en ce qu'il peut être relié à une pièce textile, par exemple un élément de vêtement ou une literie, et en ce qu'il porte une substance indicatrice qui provoque un changement de couleur lors d'une réaction avec une substance caractéristique de la transpiration humaine.

8. Dispositif selon la revendication 7, caractérisé en ce que la substance indicatrice ou les substances indicatrices sont aptes à montrer un changement de couleur lors d'une réaction avec de l'urée.

9. Dispositif selon la revendication 8, caractérisé par un mélange d'uréase et d'un indicateur de la valeur du pH comme substances indicatrices, et par de la phtaléine du phénol ou du tournesol comme substance indicatrice de la valeur du pH.

10. Dispositif selon l'une des revendications 5 à 9, caractérisé en ce que la substance indicatrice ou les substances indicatrices sont incluses sous forme de microcapsules qui peut être détruites par pression.
